(19) Europäisches Patentamt
European Patent Office
Office européen des brevets

(11) Publication number: **0 323 416 A2**

# (12) EUROPEAN PATENT APPLICATION

(21) Application number: **88830562.0**

(22) Date of filing: **22.12.88**

(51) Int. Cl.[4]: **C 07 C 143/76**
A 61 K 31/18

(30) Priority: **24.12.87 IT 4875087**

(43) Date of publication of application:
**05.07.89 Bulletin 89/27**

(84) Designated Contracting States:
**AT BE CH DE ES FR GB GR LI LU NL SE**

(71) Applicant: **Sigma-Tau Industrie Farmaceutiche Riunite S.p.A.**
**47, Viale Shakespeare**
**I-00144 Rome (IT)**

(72) Inventor: **Bagolini, Carlo Alberto**
**Via Monte Faraone, 45**
**I-00141 Roma (IT)**

**Pacifici, Licia**
**Via Saffo, 10**
**I-00125 Roma (IT)**

**Quaresima, Emma Teresa**
**Via Cratete di Mallo, 36**
**I-00124 Roma (IT)**

(74) Representative: **Fassi, Aldo, Dr.**
**c/o Sigma-Tau Industrie Farmaceutiche Riunite S.p.A.**
**Via Pontina, Km. 30, 400**
**I-00040 Pomezia (Roma) (IT)**

Claims for the following Contracting States: ES + GR.

(54) **Phenylbenzylidene derivatives of 3-aminopropanesulfonic acid having anticonvulsant activity and pharmaceutical compositions containing same for the therapeutical treatment of epilepsy.**

(57) Phenylbenzylidene derivatives of 3-aminopropanesulfonic acid having general formula

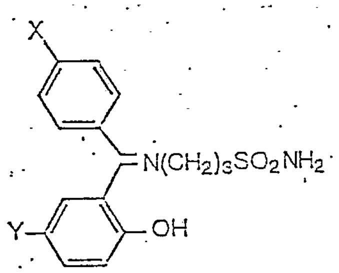

wherein X and Y, either the same or different, are hydrogen or halogen, are endowed with potent anticonvulsant activity.

EP 0 323 416 A2

## Description

The present invention relates to novel 3-APS (3-aminopropanesulfonic acid) derivatives having anticonvulsant activity and the pharmaceutical compositions containing same for the therapeutical treatment of epilepsy.

More specifically, the compounds of the present invention are phenylbenzylidene derivatives of 3-aminopropanesulfonic acid having general formula (I)

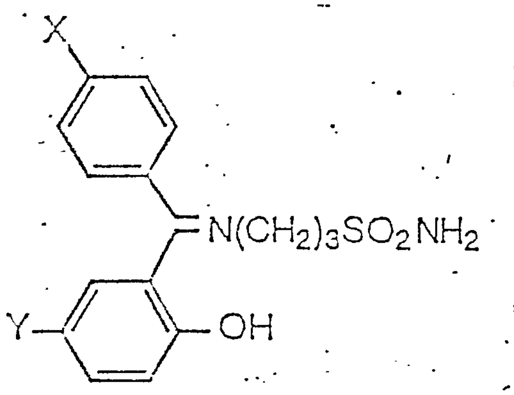

(I)

wherein X and Y, either the same or different, are hydrogen or halogen preferably chlorine and fluorine.

The potent antiepileptic activity of 3-APS in some test models has long been known. 3-APS is structurally related to both GABA (gamma-aminobutyric acid) and taurine and possesses chemical electrophysiological and biochemical properties strikingly similar to those of GABA and GABA-agonists.

It has furthermore been shown that compounds capable of increasing GABA concentration in brain by blocking its enzyme degradation, possess antiepileptic activity. GABA itself has successfully been used in some epilepsy cases. However, neither GABA nor 3-APS easily cross the blood-brain barrier and consequently do not enter the central nervous system to any appreciable degree following oral or parenteral administration.

It has been found that the 3-APS derivatives of the present invention are capable of inducing an anticonvulsive effect more potent than that of 3-APS when they are administered via the usual oral or parenteral routes.

It is interesting to note that the stucture of compounds of general formula (I) might, at first sight, appear similar to that of progabide, i.e. 4-[[α-(p-chlorophenyl)-5-fluoro-2-hydroxybenzylidene]amino]butyramide, whose antiepileptic action is well known. It should be noted, however, that this apparent resemblance is only superficial insofar as progabide is a butyric acid derivative, whereas the compounds of the present invention are derivatives of 3-aminopropanesulfonic acid.

The compounds of general formula (I) can be prepared according to the following reaction scheme wherein the main step is the 3-aminopropanesulfonamide (VII) condensation with o-hydroxybenzophenone (VI). In the following reaction scheme, X = Cl and Y = F (see Example 1).

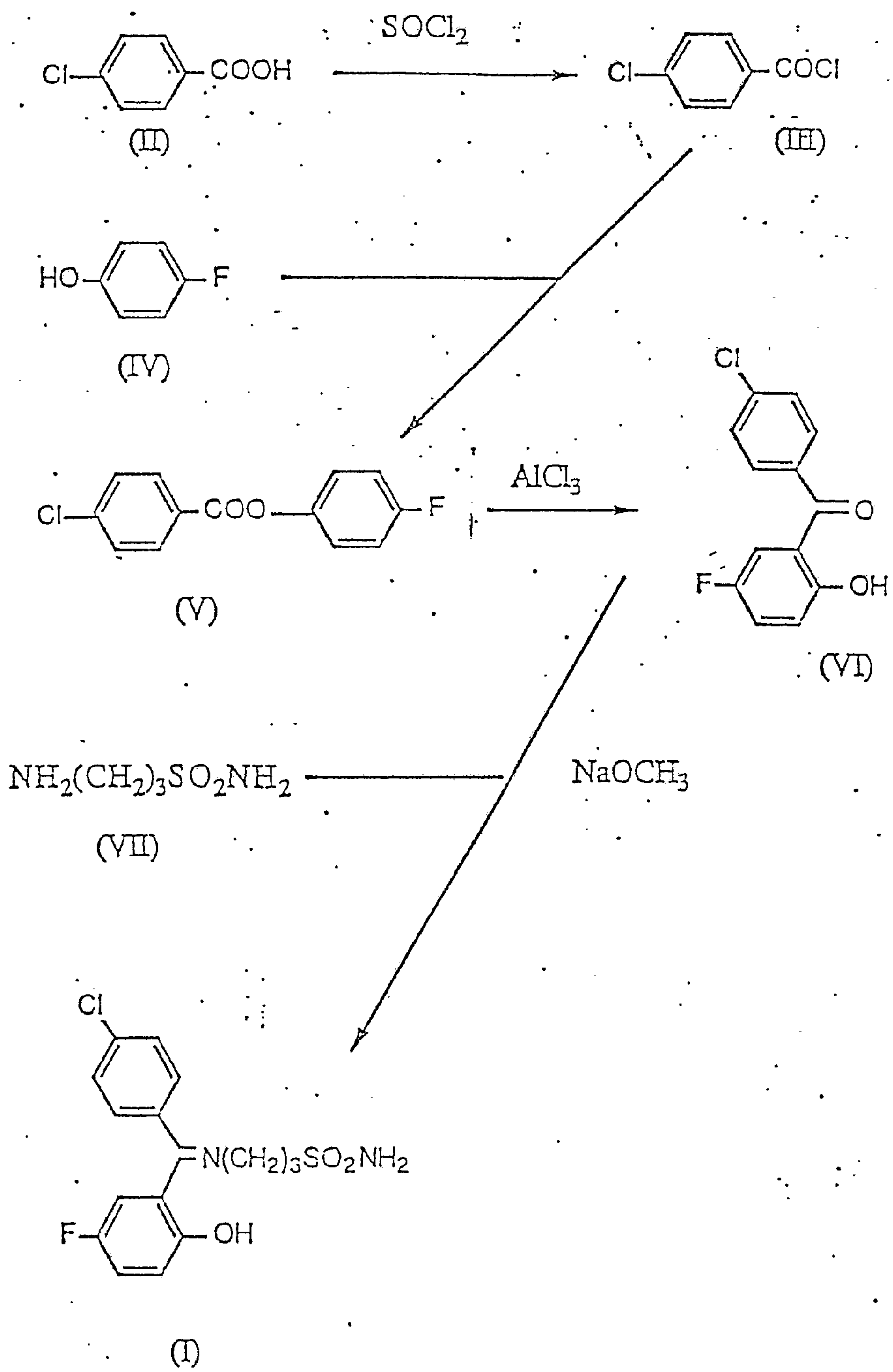


(II) (III) (IV) (V) (VI) (VII) (I)

The chlorination of p-chlorobenzoic acid (II) with $SOCl_2$ gives p-chlorobenzoyl chloride (III) which is esterified with 4-fluorophenol (IV) affording 4-fluoro-phenyl-4-chlorobenzoate (V). The isomerization of (V) with $AlCl_3$ gives 4-chloro-2′-hydroxy-5′-fluoro-benzophenone (VI) which is condensed with 3-amino-propanesulfonamide (VII) thus giving the compound (I) of the invention.

The preparation of compounds according to the invention is illustrated in the following non-limiting examples.

## Example 1

Preparation of 3-[[α-(p-chlorophenyl)-5-fluoro-2-hydroxybenzylidene]amino]propanesulfonamide (ST 505).

To a solution of 0.53 g (0.023 moles) of metal sodium in 230 milliliters of methanol, 4.3 g (0.023 moles) of 3-aminopropanesulfonamide hydrochloride and 6.25 g (0.025 moles) of 4-chloro-2′-hydroxy-5′-fluorobenzophenone were added. The reaction mixture was concentrated to dryness under vacuum at 60° C. The residue was taken up with 150 milliliters of absolute ethanol and again evaporated. This procedure was repeated four times. The residue was then taken up with ethyl acetate, filtered (to remove any insoluble material consisting of salts and starting material) and the filtered solution was washed with water (until disappearance of impurities that at TLC appeared close to the base-line), then dried over anhydrous $Na_2SO_4$ and evaporated. The raw product was crystallized from ether-hexane, thus furnishing 6 grams of the title compound;
yield 65%.
M.P. 140-142° C
TLC (AcOEt: hexane 70:30)
Anal.($C_{16}$ $H_{16}$ Cl F $N_2$ $O_3S$) C,H,Cl,F,N,S
NMR ($CDCl_3$) $\delta$ = 2.03-2.43 (2H, m, C-$CH_2$-C); 3.06-3.57 (4H, m,S-$CH_2$ e = N-$CH_2$-)

6,33-6.56 (1H, m, 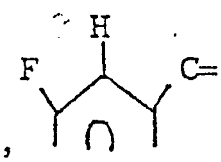 );6,87-7.77 (6H, m, Arom.)

## Example 2

Preparation of 3-[(α-phenyl)-2-hydroxybenzylidene] amino propanesulfonamide (ST 512).

3-amino-1-propanesulfonamide hydrochloride (4g; 0.023 moles) and 2-hydroxybenzophenone (5 g; 0.025 moles) were dissolved in 230 milliliters of methanol containing 0.55 grams of sodium. The solution was evaporated under vacuum taking care not to exceed 60° C. The residue thus obtained was taken up with 150 milliliters of ethanol and the solution again concentrated under vacuum. This procedure was repeated four times. The residue was dissolved in ethyl acetate and the solution washed with water. The organic phase was dried over anhydrous $Na_2SO_4$ and concentrated under vacuum. The residue was crystallized with ethyl acetate-hexane. 4.8 grams of the title compound were thus obtained; yield 60%.
M.P. 122-123° C
TLC ethylacetate: hexane 80:20, Rf 0.7
NMR $CDCl_3$ $\delta$ 7.7-6.5 (9H,m,aromatic);
3.7-3.0(4H,m,-$CH_2$ -$SO_2$-;-N-$CH_2$-); 2.5-1.9(2H,m,-$CH_2$-).

## PHARMACOLOGICAL TESTS

Tolerability

The tolerability test was carried out on male albino Swiss mice weighing 22-24 g. The animals, which had been kept fasting for 18 hours, were orally administered a suspension in 10% gum arabic of the compounds ST 505 and ST 512. The compounds were well tolerated at the dose of 3 g/kg.

Convulsant tests and antiepileptic activity.

Anticonvulsant tests were carried out using cardiazol, strychnine, bicuculline and picrotoxin. Furthermore, the antiepileptic activity was assessed via a $FeCl_3$ -induced experimental epilepsya model and sodium penicillin G-induced experimental epilepsya model.

(A) Tests on cardiazol-,strychnine-,bicuculline- and picrotoxin-induced convulsions.

The doses of cardiazol, strychinine, bicuculline and picrotoxin (100 mg/10 ml/kg i.p., 0.73 mg/10 ml/kg s.c., 2.38 mg/10 ml/kg s.c. and 4.42 mg/10 ml/kg s.c. respectively) were chosen in such a way as to provoke death of 50% of the control animals.

The tests were carried out in male albino Swiss (Charles River) mice weighing 22-24 g which had been kept fasting for 18 hours.

10% gum arabic suspensions of the compounds under examination were orally administered 60 minutes before the convulsant agent administration.

As reference substance, 3-APS (200-400-800 mg/10 ml/kg i.p.) was used.

As activity parameters the following were considered: latency time of first convulsion, percentage of animals exhibiting convulsions, death latency time and death rate.

The test results relating to 3-APS are illustrated in Table 1. 3-APS at doses of 200, 400 and 800 mg/10 ml/kg i.p. did not preserve animals from cardiazol-, strychnine-, bicuculline and picrotoxin- induced death.

At 400 mg/10 ml/kg the latency time of bicuculline-induced death and picrotoxin-induced first convulsion latency time significantly increased, while at 800 mg/kg the bicuculline-induced first convulsion latency time significantly increased.

Table 1

3-APS activity on cardiazol-, strychnine-, bicuculline and picrotoxin-induced convulsions.

| Compound | Doses mg/kg i.p. | Strong % Conv. | Cardiazol 1st conv. latency time (min.) | % Death rate | Death latency time (min.) | Strong % Conv. | Strychnine 1st conv. latency time (min.) | % Death rate | Death latency time (min.) |
|---|---|---|---|---|---|---|---|---|---|
| $H_2O$ | 10 ml. | 90 | 1′24″ ±0,31 | 50 | 20′00″ ±10,77 | 100 | 5′24″ ±0,69 | 100 | 8′06″ ±0,20 |
| 3-APS | 200 | 100 | 1′42″ ±0,26 | 50 | 4′24″ ±1,12 | 100 | 6′00″ ±0,53 | 90 | 6′43″ ±0,52 |
| $H_2O$ | 10 ml. | 100 | 1′36″ ±0,40 | 70 | 19′17″ ±6,34 | 100 | 4′00″ ±0,21 | 100 | 4′54″ ±0,57 |
| 3-APS | 400 | 100 | 1′48″ ±0,70 | 90 | 9′53″ ±2,45 | 100 | 4′00″ ±0,39 | 100 | 6′23″ ±0,68 |
| $H_2O$ | 10 ml. | 100 | 1′36″ ±0,40 | 70 | 19′17″ ±6,34 | 100 | 5′30″ ±0,45 | 80 | 9′38″ ±1,15 |
| 3-APS | 800 | 100 | 3′24″ ±0,98 | 60 | 15′40″ ±4,51 | 100 | 5′54″ ±0,50 | 90 | 10′27″ ±1,67 |
| Compound 3-APS | Doses mg/kg i.p. | Strong % Conv. | Bicuculline 1st conv. latency time (min.) | % Death rate | Death latency time (min.) | Strong % Conv. | Picrotoxin 1st conv. latency time (min.) | % Death rate | Death latency time (min.) |
| $H_2O$ | 10 ml. | 100 | 5′54″ ±0,43 | 80 | 6′53″ ±1,03 | 100 | 11′06″ ±0,35 | 30 | 32′00″ ±5,51 |
| 3-APS | 200 | 100 | 6′48″ ±0,39 | 100 | 7′30″ ±0,75 | 100 | 11′48″ ±0,44 | 20 | 56′30″ ±4,50 |
| $H_2O$ | 10 ml. | 88* | 6′47″ ±0,60 | 75 | 6,33″ ±0,21 | 100 | 10′30″ ±0,69 | 50 | 30′00″ ±2,17 |
| 3-APS | 400 | 100* | 7′29″ ±0,56 | 75 | 7′40″ ±0,33 | 100 | 13′00″ ±0,68 | 30 | 29′34″ ±4,18 |
| $H_2O$ | 10 ml. | 80 | 6′53″ ±0,63 | 40 | 9′30″ ±2,78 | 100 | 11′42″ ±0,45 | 30 | 48′40″ ±9,84 |
| 3-APS | 800 | 70 | 9′08″ ±0,48 | 40 | 9′30″ ±0,65 | 30 | 11′48″ ±0,39 | 70 | 52′34″ ±13,34 |

CONV. = CONVULSIONS.

10 animals per group (   = 8 animals per group.) were used.

Student's "t" test.   and Δ indicate P = 0.02 and P = 0.01 respectively with reference to the control group.

(′ means minutes. ″ means seconds)

The test results relating to the compounds under examination are illustrated in Tables 2 and 3.

ST 512, administered at the doses equimolar to 100 and 200 mg/10ml/kg of 3-APS reduced by 20 and 10%, respectively, the cardiazol-induced death rate.

ST 505 was orally administered to male albino Swiss rats weighing 22-24 g at the doses equimolar to 100 and 200 mg/10ml/kg of 3-APS, 60 minutes before convulsant administration.

The dose of 100 mg/kg (see table 3) significantly increased the latency time of the bicuculline-induced first convulsion and death and reduced by 20% the strychnine- and bicuculline-induced death rate, it did not change cardiazole and picrotoxin response. The dose of 200 mg/kg preserved all the animals from strychnine-induced convulsions and death; it, furthermore, increased the latency time of cardiazole-bicuculline-, picrotoxin-induced first convulsion and bicuculline-induced death. The compound was also orally administered to male albino Wistar rats weighing 170-200 g (see table 3), at the dose equimolar to 100 mg 3-APS 60 minutes before cardiazole administration (70 mg/10ml/kg i.p.). This test showed a 30% death rate reduction and a significant increase in the first convulsion latency time.

Table 2

Activity of ST 512 administered orally on the cardiazol-, strychnine-, bicuculline and picrotoxin induced convulsions.

| Compounds | Doses mg/kg eq. 3 APS | Strong % Conv. | Cardiazol 1st conv. latency time (min.) | % Death rate | Death latency time (min.) | Strong % Conv. | Strychnine 1st conv. latency time (min.) | % Death rate | Death latency time (min.) |
|---|---|---|---|---|---|---|---|---|---|
| $H_2O$ | 10 ml | 100 | 1'18" ±0,21 | 70 | 10'37" ±2,03 | 80 | 7'42" ±0,67 | 80 | 7'45" ±0,67 |
| ST 512 | 100 | 100 | 1'24" ±0,27 | 50 | 11'00" ±5,11 | 90 | 6'06" ±0,46 | 80 | 7'00" ±0,89 |
| $H_2O$ | 10 ml | 100 | 1'18" ±0,21 | 70 | 10'37" ±2,03 | 80 | 7'42" ±0,67 | 80 | 8'45" ±0,67 |
| ST 512 | 200 | 100 | 1'42" ±0,30 | 60 | 10'20" ±2,49 | 80 | 7'18" ±0,47 | 90 | 8'53" ±0,51 |
| Compounds | Doses mg/kg eq. 3 APS | Strong % Conv. | Bicuculline 1st conv. latency time (min.) | % Death rate | Death latency time (min.) | Strong % Conv. | Picrotoxin 1st conv. latency time (min.) | % Death rate | Death latency time (min.) |
| $H_2O$ | 10ml | 100 | 6'00" ±0,37 | 100 | 8'303±0,65 | 100 | 12'38" ±0,69 | 40 | 22'45" ±4,37 |
| ST 512 | 100 | 90 | 7'12" ±0,93 | 100 | 9'18" ±0,61 | 100 | 13'42" ±0,73 | 40 | 31'30" ±2,25 |
| $H_2O$ | 10 ml | 100 | 5'54" ±0,57 | 100 | 8'18" ±0,45 | 100 | 12'30" ±0,69 | 40 | 22'45" ±4,37 |
| ST 512 | 200 | 100 | 6'12" ±0,92 | 100 | 7'48" ±0,86 | 100 | 13'06" ±0,91 | 50 | 31'00" ±1,55 |

Table 3

Activity of ST 505 administered orally on the cardiazol-, strychnine-, bicuculline- and picrotoxin-induced convulsions.

| COMPOUNDS | DOSES mg/kg eq. 3 APS | CARDIAZOL Strong % conv. | 1st conv. latency time (min.) | % Death rate | Death latency (min.) | STRYCHNINE Strong % conv. | 1st conv. latency time (min.) | % Death rate | Death laten (min.) |
|---|---|---|---|---|---|---|---|---|---|
| GUM ARABIC | 10 ml | 100 | 0'54"±0,23 | 40 | 17'00"±6,01 | 70 | 7'08"±0,95 | 50 | 7'36"±0,68 |
| ST 505 | 100 | 90 | 3'42"±1,94 | 90 | 13'00"±3,77 | 60 | 9'20"±1,28 | 30 | 8'09"±2,19 |
| ST 505 | 200 | 80 | 5'42"±2,05 | 40 | 13'20"9,94 | 0 | == | 0 | === |
| GUM ARABIC (RAT) | 10 ml | 90 | 1'07"±0,11 | 90 | 4'20"±0,11 | | | | |
| ST 505 (RAT) | 100 | 60 | 1'40"±0,17 | 60 | 7'10"±2,77 | | | | |

Table 3 (con.t)

| COMPOUNDS | DOSES mg/kg eq. 3-APS | BICUCULLINE Strong % conv. | 1st conv. latency time (min.) | % Death rate | Death latency (min.) | PICROTOXIN Strong % conv. | Death latency (min.) | % Death rate | Death latency (min.) |
|---|---|---|---|---|---|---|---|---|---|
| GUM ARABIC | 10 ml | 100 | 4'18"±0,26 | 100 | 7'06"±1,21 | 100 | 12'12"±0,53 | 70 | 35'17"±3,35 |
| ST 505 | 100 | 100 | 7'48"±0,83 ▲ | 80 | 11'30"±1,49 ■ | 100 | 11'54"±0,60 | 90 | 31'53"±2,76 |
| ST 505 | 200 | 100 | 5'18"±0,30 ■ | 80 | 12'15"±0,86 △ | 100 | 15'42"±0,70 ▲ | 90 | 40'40"±4,11 |
| GUM ARABIC (RAT) | 10 ml | | | | | | | | |
| ST 505 (RAT) | 100 | | | | | | | | |

(B) Antiepileptic activity test ($FeCl_3$-induced epilepsy model.

Male albino Wistar rats weighing 250-300 g were used. The animals, anaesthetized with Nembutal Na (30 mg/kg i.p.), were stereotactically injected in their left ventral hippocampus one microliter of 0.55 M $FeCl_3$ solution (AP = -3.2; L = 5.0.; H = 5.5 Pellegrino and Cushman, 1971). Ten minutes following injection, recording electrodes were implanted: in both ventral hippocampi, two millimiters below the $FeCl_3$ injection site (bipolars), bilaterally on the frontoparietal cortex (monopolars). recording of cerebral bioelectric activity, carried out with an EEG OTE-Biomedica PF 14e recorder, began about one hour following $FeCl_3$ injection and lasted more than three hours on end.The epilepsy degree was assessed by calculating for each animal the number and duration in seconds of the seizures at 15-minute intervals during the whole recording span both in the hyppocampus where the injection had been performed and in the contralateral cortex. Moreover, an "index of epileptization" was calculated as the mean product of seizure number times the duration of the seizures in each time interval. 500, 250 and 125 mg/kg of the compound under examination, suspended in gum arabic, were orally administered 45 minutes following $FeCl_3$ injection.

Two groups of control animals received, at the same time and via the same administration route as the test animals, an identical volume of vehicle.

The results thus obtained show the antiepileptic activity of the compound assessed by examining the whole phenomenon as it evolves in time via the measure (carried out with the trapezoidal rule) of the area under the curve wherein the average number of seizures and their duration is plotted versus time.

By comparing the areas relating to the controls with the areas relating to the treated animals via Mann-Whitney's "U" test, it was assessed that ST 505, at the dose of 500 mg/kg and ST 521 at the dose of 250 mg/kg, presented a statistically significant difference ($P < 0.05$) with respect to the controls.

(C) Auricular electroshock.

Male albino Swiss mice weighing 22-24 g were used in this test. The electroshock conditions were the shock duration: 3 seconds; frequence: 100; pulse amplitude: 0.6 milliseconds, current intensity: 40 milliamperes.

The compounds ST 505 and ST 512 suspended in gum arabic were orally administered 60 minutes before the electroshock.

The tests results which illustrate the number of animal preserved from death provoked by maximal electroshock are shown in the following Table 4.

TABLE 4

Protective effect of ST 505 and ST 512 against auricular electroshock

| Compound | dose | number of animals | death rate |
|---|---|---|---|
| Gum arabic | 10 ml/kg | 10 | 6/10 |
| ST 505 | 200 | 10 | 4/10 |
| ST 505 | 400 | 10 | 5/10 |
| Gum arabic | 10 ml/kg | 10 | 7/10 |
| ST 512 | 200 | 10 | 3/10 |
| ST 512 | 400 | 10 | 4/10 |

(D) Sodium penicillin G-induced epilepsy

In this regard, see: Holmes, O. and Lockton, J.W.: "Locally generated interictal epilectic spikes in the cortex cerebri of the rat". J.Physiol. (Lond.), 308 (1980) 75-76 P. Lokcton, J.W. and Holmes, O: "Site of penicillin-induced epilepsy in the cortex cerebri of the rat'. Brain research, 190 (1980) 301-304. Reichental, E. and Hocherman, S. : "The critical cortical area for development of penicillin-induced epilepsy". Electroenceph. Clin. Neurophysiol., 42 (1977) 248-251).

Male albino Wistar rats weighing 260-280 g were used in this test. Epilepsy was induced in these rats through the application of 5 mg of sodium penicillin G, power (Carlo Erba) on the left cortex cerebri. EEG recording (electroencephalograph OTE-Biomedica PF 14e) was carried out with cortical electrodes. Following a 15 minute recording period (basal), the animals were orally administered 250 mg/10 ml/kg of ST 505 suspended in 10% gum arabic. The controls were administered 10 ml/kg of 10% gum arabic.

The following parameters were taken into account:

a) number of peaks of the EEG tracing in subsequent 15 minu te intervals, in an overall 5-hour recording period; and

b) number of fits with attendant average duration for each interval.

With regard to parameter a), the area under the curve was calculated wherein the number of peaks at different times and its accumulation function were plotted versus the observation time, thus obtaining five descriptive parameters: one which gives the measure (area) of the totale amount of epilepsy and four (shape indexes) describing the trends of the peaks during the time interval (five hours) under examination. These shape indexes were the percentile values at 50%, 60%, 70% and 80% of the accumulation function of the area versus time and represented, therefore, for each rat, a measure of the distribution of the epileptic fits in time.

The fits were studied with the same model as that utilized for the peaks.

Table 5 shows that ST 505 has significantly decreased the area of the peaks, thus evidencing ST 505 antiepileptic activity (Mann Whitney's U test).

Table 5

| | CONTROLS MEAN (S.E.) | TREATED WITH ST 505 (250 mg/kg) MEAN (S.E.) |
|---|---|---|
| Peak area | 77.8 (9.54) | 52.9 (5.87)** |
| Ist shape index | 6.7 (0.48) | 8.5 (1.0)** |
| 2nd shape index | 8.9 (0.64) | 9.8 (1.2) |
| 3rd shape index | 10.8 (0.65) | 11.8 (1.4) |
| 4th shape index | 12.5 (0.69) | 13.8 (1.4) |

** = $P < 0.05$ (Mann Whitney's "U" test)

Approximately 20-100 mg of a compound of general formula (I) kg of body weight/day were found to be the appropriate dose for oral administration, although lower or higher doses, based on sound physician's judment and having regard to patient's age, weight general and pathological conditions can be administered.

In practice, the compounds are orally or parenterally administered in any one of the usual pharmaceutical formulations which are prepared according to conventional procedures well-known to those skilled in pharmaceutical technology. These formulations comprise solid and liquid unit dosage forms, such as tablets, capsules and injectables forms, e.g. sterile solutions for ampules and vials.

## Claims

1. Compound of general formula

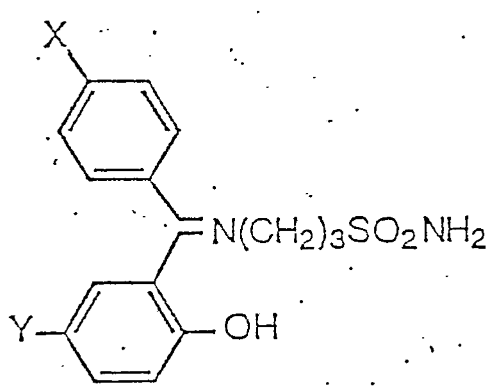

(I)

wherein X and Y, either the same or different, are hydrogen or halogen.

2. The compound of claim 1, wherein X = Y = H
3. The compound of claim 1, wherein X = Cl and Y = F.
4. An orally or parenterally administrable pharmaceutical composition for treating epilepsy comprising

an amount therapeutically effective for inducing an anticonvulsant effect in an epileptic patient of a compound of general formula (I) and a pharmaceutically acceptable excipient.

5. The composition of claim 4, wherein the compound is the compound of claim 2.

6. The composition of claim 4, wherein the compound is the compound of claim 3.

**Claim for the following Contracting States: GR, ES**

A process for producing a compound of general formula (I)

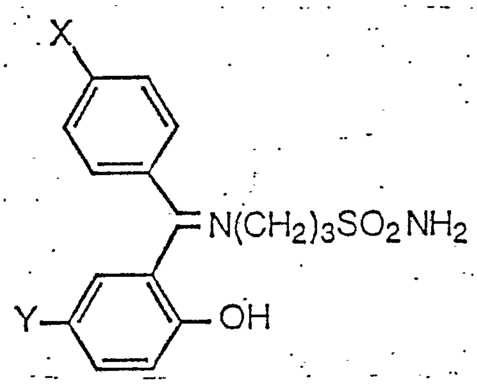

(I)

according to the following reaction scheme:

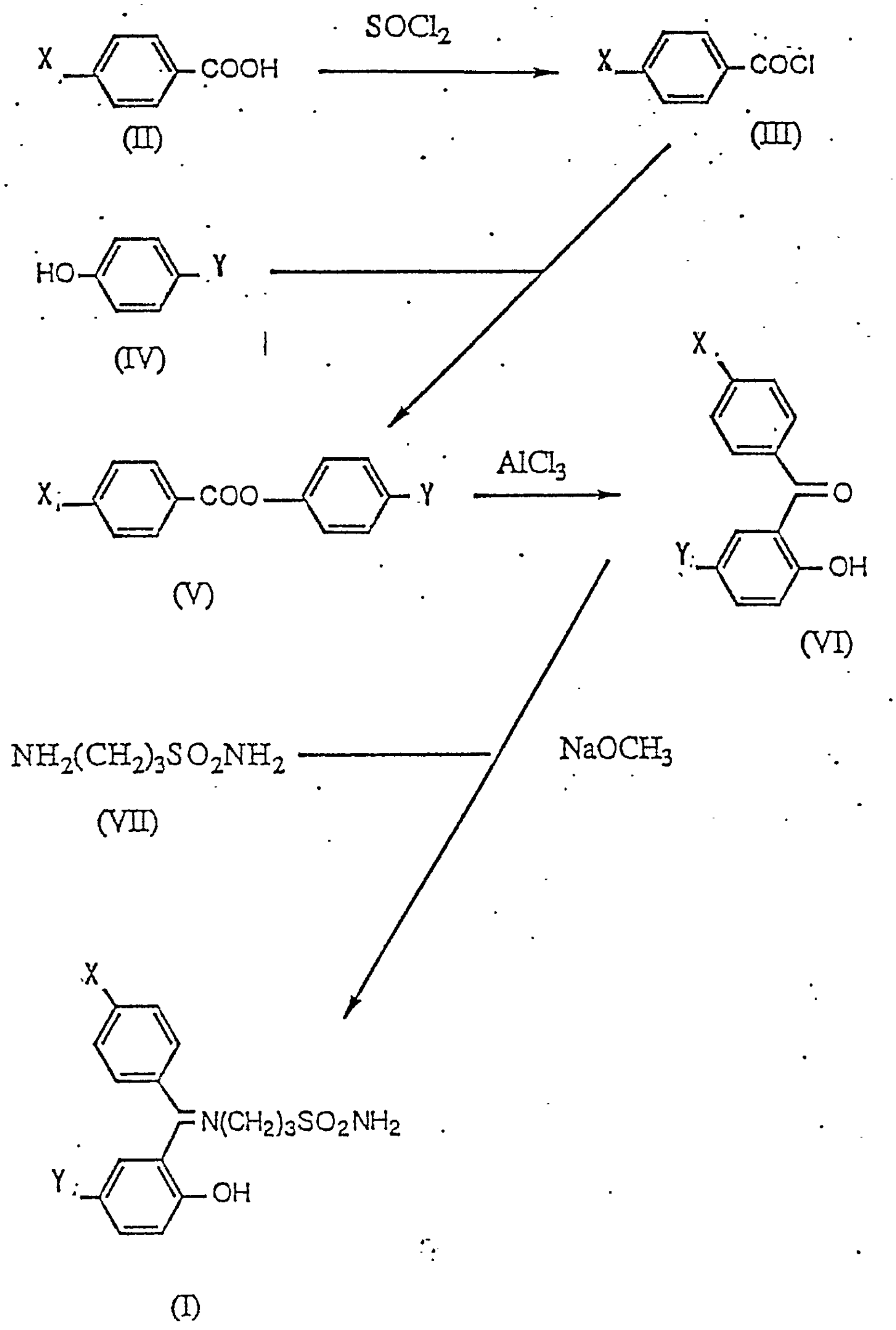


which comprises:

(a) chlorinating (II) to (III) with thionyl chloride;
(b) esterifying (III) with (IV), thus obtaining (V);
(c) isomerizing (V) in the presence of $AlCl_3$, thus obtaining (VI); and
(d) condensing (VI) with 3-aminopropanesulfonamide (VII) in the presence of sodium methoxide.